# EUROPEAN PATENT SPECIFICATION

(11) **EP 3 199 198 B1**
(45) Date of publication and mention of the grant of the patent: **14.12.2022**
(21) Application number: 15843923.2
(22) Date of filing: 27.08.2015
(51) Int. Cl.: A61M 25/09

(54) **GUIDE WIRE**
FÜHRUNGSDRAHT
FIL-GUIDE

(30) Priority: 26.09.2014 JP 2014196648
(43) Date of publication of application: 02.08.2017
(73) Proprietor: Terumo Kabushiki Kaisha, Tokyo 151-0072 (JP)
(72) Inventor: OOTANI Yasunao, Fujinomiya-shi Shizuoka 418-0015 (JP); TANO Yutaka, Fujinomiya-shi Shizuoka 418-0015 (JP); INOUE Shuuhei, Fujinomiya-shi Shizuoka 418-0015 (JP); FUJIKI Jo, Fujinomiya-shi Shizuoka 418-0015 (JP); KAMBARA Kayo, Fujinomiya-shi Shizuoka 418-0015 (JP)
(74) Representative: Casalonga
(86) International application number: PCT/JP2015/074302
(87) International publication number: WO 2016/047364

(56) References cited:
- JP-A- 2005 534 407
- JP-A- 2011 110 384
- US-A- 5 406 960
- US-A- 5 497 785
- US-A1- 2009 157 050

## Description

### Technical Field

The present invention relates to a guide wire used when guiding a catheter into a lumen in a living body, in particular, a blood vessel, In particular, the present invention relates to a guide wire according to the premable of claim 1, such as it is e.g. known from US5,406,960A.

### Background Art

The guidewire is used when guiding a catheter which is used for treatment of a site in which it is difficult to perform a surgical operation, for example, percutaneous transluminal coronary angioplasty (PTCA) or treatment which is aimed to be less invasive to the human body, or used in tests such as cardioangiography, into a blood vessel. PTCA is a treatment method for dilating a stenosed site of a coronary artery with a balloon or the like to secure a blood flow path.

In PTCA, a balloon catheter is guided to a stenosed site by inserting a guide wire into the vicinity of the stenosed site of a blood vessel in a state in which a distal portion of the guide wire protrudes from a distal portion of the balloon catheter. At that time, it is necessary for the guide wire to select and pass through a meandering or bifurcated blood vessel, a stenosed blood vessel, or the like. In addition, it is necessary to widen or penetrate deposits such as cholesterol constituting the stenosed site using a pushing force of the guide wire in the stenosed site. Accordingly, excellent flexibility (blood vessel followability) for following the shape of a blood vessel and for preventing damage to a blood vessel wall, and excellent pushing performance (pushability) which ensure effective transmission of a pushing force from the operator's hand side (proximal portion) to a distal portion are required for the guide wire used for PTCA.

In addition, in PTCA, in some cases, reshaping at a distal end is performed before inserting the guide wire into the blood vessel in order to make the guide wire follow the bent and bifurcated blood vessel. Specifically, a surgeon performs the reshaping by bending the distal portion of the guide wire into a predetermined shape (for example, J shape) using fingers in accordance with the shape of the bifurcated blood vessel or the like. Accordingly, it is necessary for the guide wire to easily perform such reshaping at a distal end.

In the related art, a guide wire including the following configuration has been proposed in Patent Literature 1 as a guide wire used for PTCA. The guide wire in Patent Literature 1 includes a core portion formed of an elongated object; and a coil which is provided so as to cover a distal side of the core portion. The core portion has a flat plate portion, which is formed to have a plate width equal to or more than twice the plate height (plate thickness), on the distal side.

### Citation List

### Patent Literature

Patent Literature 1: International Publication No. 2009/126656

### Summary of Invention

### Problem to be Solved by Invention

In the guide wire of Patent Literature 1, the distal portion of the guide wire becomes flexible because a flat plate portion with a thin plate thickness is provided on the distal side of the core portion, and it is expected to improve safety and blood vessel followability to some degree. However, the guide wire in Patent Literature 1 includes a round rod-like main body portion which is formed on a proximal side and has a circular shape in transverse cross section; a flat plate portion which is formed on a distal side and has a rectangular shape in transverse cross section; and a transition portion which connects the main body portion and the flat plate portion. Accordingly, in the guide wire of Patent Literature 1, the transverse cross sectional shape greatly changes from the transition portion over the flat plate portion. Therefore, the physical properties (in particular, rigidity) also greatly change from the transition portion over the flat plate portion.

In such a guide wire, when the proximal portion of the guide wire is rotated in order to make the guide wire pass a meandering or bifurcated blood vessel, the flat plate portion is twisted or is buckled in the vicinity of a boundary between the transition portion and the flat plate portion. As a result, rotary torque at the proximal portion of the guide wire is not effectively transmitted from the proximal portion to the distal portion. Therefore, there is a problem in that the distal portion of the guide wire does not face an intended direction and blood vessel followability of the guide wire is decreased. In addition, there is a problem in that torquability is decreased due to twisting of the flat plate portion when the proximal portion of the guide wire is rotated in order to advance the guide wire in a stenosed site, or that pushability and trackability (properties of transmitting a rotational force, which is applied to the guide wire at the proximal portion, to the distal portion) of the guide wire without effective transmission of a pushing force of the proximal portion of the guide wire to the distal portion due to buckling of the flat plate portion in the vicinity of a boundary between the transition portion and the flat plate portion.

The present invention has been devised in order to solve such problems. Therefore, an object of the present invention is to provide a guide wire having excellent blood vessel followability, pushability, and trackability.

### Means to Solve Problem

In order to solve the problems, there is provided a guide wire according to includes a core portion an elongated object claim 1. The dependent claims relate to advantageous embodiments.

It is preferable that the guide wire according to the present invention includes a coil portion which is disposed so as to cover the distal side of the core portion and is obtained by forming strands in a spiral shape, and the core portion and the coil portion are fixed to each other on the distal side. In addition, it is preferable that the guide wire according to the present invention includes a resin covering portion which is formed so as to cover the distal side of the core portion and is made of a resin material.

According to the configuration, a portion with a thin plate thickness is disposed on the distal side of the guide wire because the flat plate portion is provided on the distal side of the core portion. Therefore, rigidity at a distal portion of the guide wire is decreased and flexibility of the guide wire at the distal portion is improved. In addition, since the rigidity at the distal portion of the guide wire is decreased, reshaping of the distal portion of the guide wire is easily performed in accordance with the shape of a bifurcated blood vessel or the like. In addition, significant change in the transverse cross sectional shape and significant change in the rigidity from the transition portion over the flat plate portion are prevented because at least one groove portion is formed in the transition portion which connects the main body portion and the flat plate portion of the core portion. Accordingly, twisting of the flat plate portion or buckling in the vicinity of a boundary between the transition portion and the flat plate portion is suppressed when the guide wire passes a meandering or bifurcated blood vessel or when the guide wire advances in a stenosed site. As a result, it is possible to effectively transmit rotary torque of a proximal portion of the guide wire to the distal portion, and therefore, it is possible to make the distal portion of the guide wire face an intended direction. In addition, it is possible to effectively transmit pushing force of the proximal portion of the guide wire to the distal portion.

In addition, in the guide wire according to the present invention, it is preferable that at least one groove portion extending in the direction different from the length direction is formed on at least an upper surface or a lower surface of the flat plate portion in the length direction.

According to the configuration, the rigidity of the flat plate portion is further decreased because the groove portion is formed in the flat plate portion. Therefore, the rigidity at the distal portion of the guide wire at which the flat plate portion is disposed is decreased and the flexibility of the guide wire at the distal portion is further improved.

### Effect of Invention

According to the guide wire of the present invention, the flexibility of the guide wire on the distal side is improved and the change in the rigidity of a distal flexible portion is decreased. Therefore blood vessel followability of the guide wire becomes excellent. In addition, since the buckling of the core portion or the like is suppressed, it is possible to effectively transmit rotary torque or pushing force at the proximal portion of the guide wire to the distal portion. Therefore, blood vessel followability, pushability, and trackability of the guide wire become excellent.

### Brief Description of Drawings

[Fig. 1] Fig. 1 is a partial longitudinal sectional view showing a first embodiment of a guide wire of the present invention.
[Fig. 2] Fig. 2 is a side view of a core portion of the guide wire on a distal side shown in Fig. 1.
[Fig. 3] Fig. 3 is a plan view of the core portion of the guide wire on the distal side shown in Fig. 1.
[Fig. 4] Fig. 4 is an end surface view of the core portion taken along line A-A shown in Fig. 3.
[Fig. 5] Fig. 5 is an end surface view of the core portion taken along line B-B shown in Fig. 3.
[Fig. 6] Fig. 6 is a side view of a core portion on a distal side which shows a second embodiment of a guide wire of the present invention.
[Fig. 7] Fig. 7 is a plan view of the core portion on the distal side shown in Fig. 6.
[Fig. 8] Fig. 8 is a partial longitudinal sectional view showing a third embodiment of a guide wire of the present invention on a distal side of the wire.

### Embodiments for Carrying out Invention

A first embodiment of a guide wire according to the present invention will be described in detail while referring to drawings. Note that, in the present invention, a distal side refers to a side on which a guide wire is inserted into a blood vessel, and a proximal side refers to a side on which a surgeon or the like operates the guide wire.

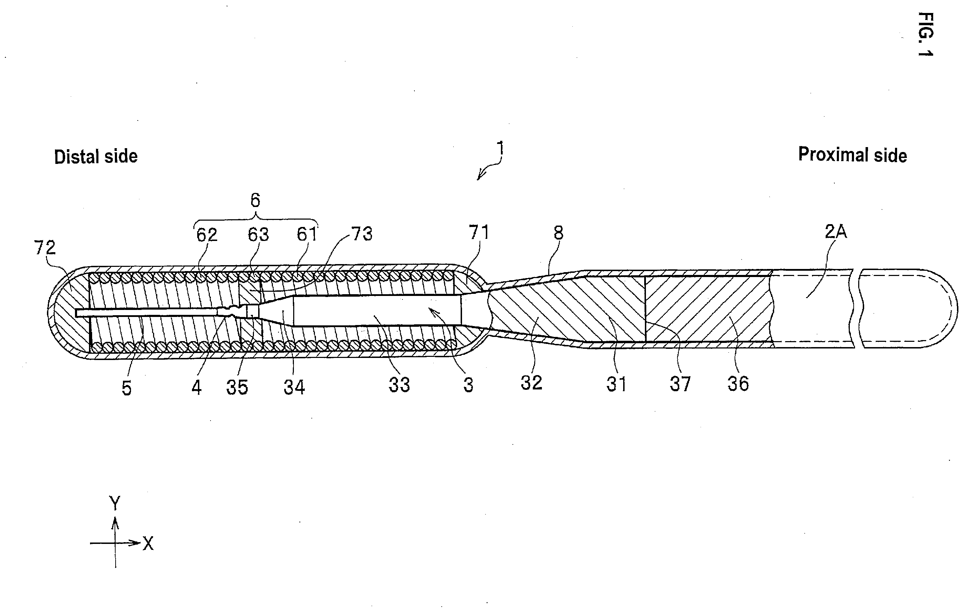

As shown in Fig. 1, a guide wire (hereinafter, referred to as a wire) 1 is an elongated object including a core portion 2A which includes a main body portion 3, a transition portion 4, and a flat plate portion 5. Groove portions 41 and 42 are formed in the transition portion 4. The total length of the wire 1 is not particularly limited, and is preferably, for example, 200 to 5000 mm. In addition, it is preferable that the wire 1 includes a coil portion 6 disposed so as to cover a distal side of the core portion 2A, and in which the core portion 2A and the coil portion 6 are fixed to each other on the distal side. As the fixing method, a fixation material (fixation portion) 72 such as solder (brazing material) or an adhesive material is preferably used for the fixing, and the fixation portion 72 may be formed through welding. Hereinafter, each configuration will be described.

### <Core Portion>

As shown in Figs. 1 to 3, the core portion 2A is formed of an elongated object having flexibility. The core portion 2A is preferably made of an elastic metal material such as Ni-Ti alloy or stainless steel in consideration of the flexibility and the strength of the wire 1. The core portion 2A sequentially includes the main body portion 3, the transition portion 4, and the flat plate portion 5 from a proximal side to the distal side, and at least one of the groove portion 41 or the groove portion 42 is formed in the transition portion 4. Note that the groove portion 42 may not be formed.

### (Main Body Portion)

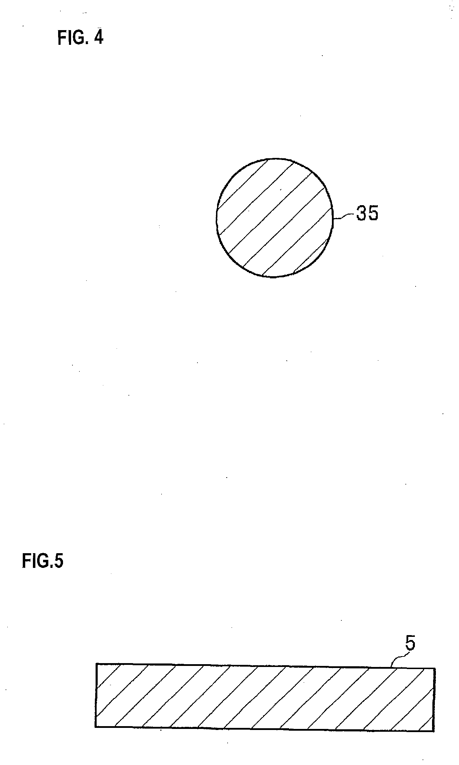

As shown in Figs. 1 to 3, the main body portion 3 is formed of an elongated object with a bar shape (non-plate shape). It is preferable that the transverse cross sectional shape (which is a YZ-axis plane and a cross section perpendicular to a length direction) of the main body portion 3 is substantially a circular shape (refer to Fig. 4). In addition, it is preferable that the main body portion 3 includes a large-diameter portion 31 having a constant outer diameter from the proximal side to the distal side; a first tapered portion 32 of which the outer diameter is decreased toward the distal side; a middle-diameter portion 33 having a constant outer diameter; a second tapered portion 34 of which the outer diameter is decreased toward the distal side; and a small-diameter portion 35 having a constant outer diameter.

Two tapered portions of the first tapered portion 32 and the second tapered portion 34 are described above as tapered portions formed between portions (between the large-diameter portion 31 and the middle-diameter portion 33, and between the middle-diameter portion 33 and the small-diameter portion 35) which have a constant diameter. However, the number of the tapered portions is not limited to two, and at least one tapered portion may be formed. In addition, a large-diameter portion 36 which has the same outer diameter as that of the large-diameter portion 31 and has a constituent material different from that of the large-diameter portion 31 may be joined to the large-diameter portion 31 in a joint portion (welded portion) 37. The joining method is not particularly limited, but examples thereof include butt resistance welding such as friction pressure welding, spot welding using a laser, or upset welding, and joining using a tubular joint member.

### (Flat Plate Portion)

As shown in Figs. 1 to 3, the flat plate portion 5 provides flexibility to the wire 1 (core portion 2A) and is formed of an elongated plate-shaped flat plate having a rectangular shape in transverse cross section (refer to Fig. 5) so as to facilitate reshaping of a distal portion of the wire at the distal end. The flat plate portion 5 preferably has a plate length of 1 to 30 mm, a plate width of 0.1 to 0.5 mm, and a plate width of 0.01 to 0.06 mm. In addition, the plate width of the flat plate portion 5 may be increased or decreased toward the distal side, and the plate thickness may also be increased or decreased toward the distal side.

In addition, the distal side of the flat plate portion 5 is fixed to the coil portion 6 using the fixation material (fixation portion) 72 or the like. In addition, the flat plate portion 5 is preferably produced together with the transition portion 4 to be described below by pressing the distal side of the bar-shaped main body portion 3, preferably the distal side of which the diameter is reduced, using a mold or the like. Note that since the transverse cross sectional shape of the flat plate portion 5 is produced through the pressing, both ends thereof are slightly rounded and have an approximately rectangular shape in transverse cross section. However, the roundness of the both ends are omitted in Fig. 5 for the convenience of description.

### (Transition Portion)

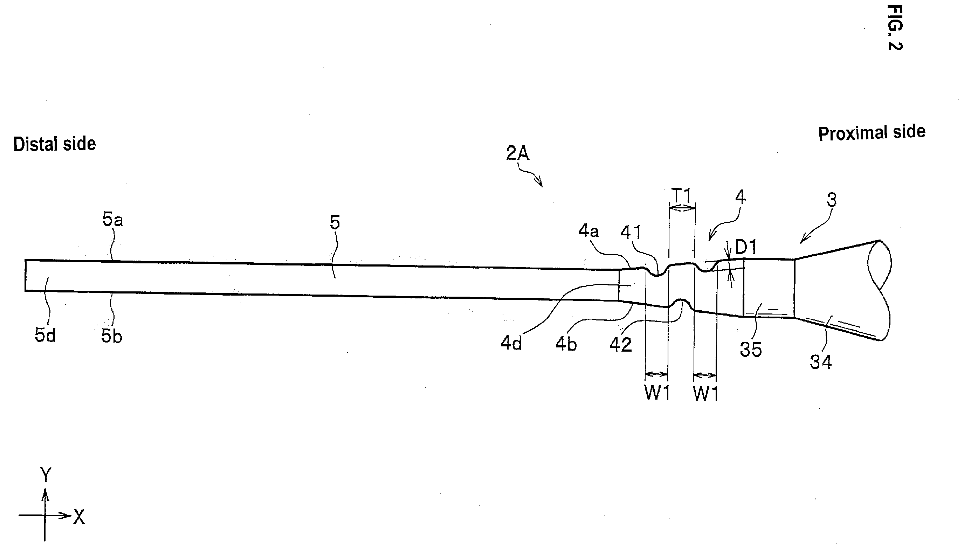

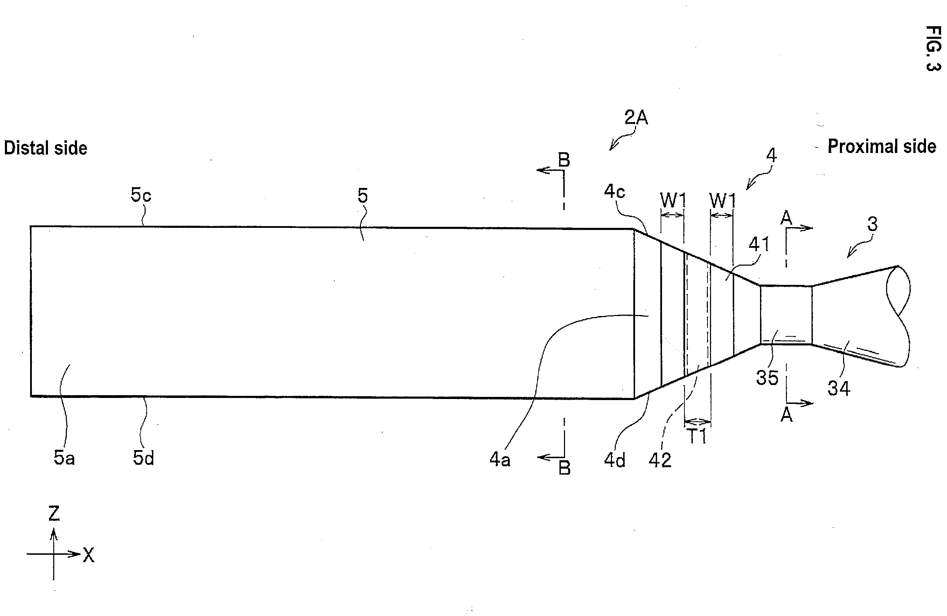

As shown in Figs. 2 and 3, the transition portion 4 is a portion which connects the main body portion 3 and the flat plate portion 5 and which is gradually changed from a circular shape in transverse cross section (refer to Fig. 4) to the rectangular shape in transverse cross section (refer to Fig. 5) from the proximal side toward the distal side. The length of the transition portion 4 is preferably 1 to 10 mm. In addition, the transition portion 4 has four slopes 4a, 4b, 4c, and 4d which are connected to surfaces of the flat plate portion 5. At least one of the groove portion 41 or the groove portion 42 extending in a direction different from the length direction is formed on at least one slope in the length direction. Note that the groove portion 42 may not be formed. The groove portions 41 and 42 are preferably produced by pressing a mold having a surface of a mold, in which convex portions with a shape similar to the groove portions 41 and 42 are formed, on the slope of the transition portion 4.

As shown in Figs. 2 and 3, the groove portion 41 is formed on the slope 4a on the same surface side as an upper surface 5a of the flat plate portion 5. Note that, although not shown in the drawing, the groove portion 41 may be formed on the slope 4b or the slopes 4c and 4d on the same surface sides as a lower surface 5b or side surfaces 5c and 5d of the flat plate portion 5. Although not shown in the drawing, the groove portion 41 may be continuously formed on at least two slopes out of peripheral surfaces consisting of the slope 4a to the slope 4d.

The direction in which the groove portion 41 is formed is not particularly limited, but is preferably a direction orthogonal to the length direction as shown in Fig. 3. Note that, although not shown in the drawing, the groove portion 41 may be formed in a direction inclined to the direction orthogonal to the length direction at a predetermined angle. Furthermore, the planar shape of the groove portion 41 is preferably a linear shape as shown in Fig. 3, but may be a polygonal line shape or a curved shape.

It is preferable that the transverse cross sectional shape of the groove portion 41 formed in the transition portion 4 is an approximately semi-circular shape. However, the transverse cross sectional shape of the groove portion 41 may be other shapes, for example, an approximately U-shape, an approximately V-shape, or an approximately rectangular shape. The number of groove portions 41 is preferably 1 to 100. The groove width W1 of the groove portion 41 is preferably 0.001 to 3 mm. The groove depth D1 of the groove portion 41 is preferably 0.005 to 0.05 mm. In addition, in a case of forming a plurality of groove portions 41, the groove width W1 is preferably formed to be constant, but may be formed so as to be increased or decreased toward the distal side. The groove depth D1 is also preferably formed to be constant, but may be formed so as to be increased or decreased toward the distal side.

An interval T1 of adjacent groove portions 41 is preferably 0.005 to 3 mm. The plurality of groove portions 41 are preferably formed at even intervals, but may be formed so that the interval T1 increases or decreases toward the distal side. In addition, the plurality of groove portions 41 may have an interval T1 of 0 mm, that is, may be continuously formed. Furthermore, the groove portions 41 which have been continuously formed may be formed in a part or all of the slopes 4a, 4b, 4c, and 4d of the transition portion 4.

As shown in Figs. 2 and 3, in the transition portion 4, it is preferable that the groove portion 41 is formed on the slope 4a and the groove portion 42 is formed on the slope 4b on the same surface side as the lower surface 5b of the flat plate portion 5. In addition, it is preferable that the groove portion 41 and the groove portion 42 are mutually alternately disposed in the length direction of the transition portion 4. However, the groove portion 41 and the groove portion 42 may be disposed at the same position as each other. In addition, although not shown in the drawing, in a case where the groove portion 41 is formed on the slope 4b, the groove portion 42 is formed on the slope 4a. In addition, although not shown in the drawing, in a case where the groove portion 41 is formed on one of the slopes 4c and 4d, the groove portion 42 is formed on one of the slopes 4d or 4c which is on a side opposite to the slope on which the groove portion 41 is formed.

Although not shown in the drawing, in a case where the groove portions 41 are continuously formed on at least two slopes on the peripheral surface consisting of the slopes 4a to 4d, the groove portions 42 may be disposed and formed alternately with the groove portion 41 on slopes on which the groove portion 41 has not been formed.

The formation direction, the planar shape, the transverse cross-sectional shape, the number of grooves, the groove width, and the groove depth of the groove portion 42, and the interval between adjacent groove portions 42 are the same as those of the groove portion 41, and therefore, the description thereof will not be repeated. In addition, the formation direction or the like of the groove portion 42 is preferably the same as that of the groove portion 41, but may be different from that of the groove portion 41.

Although not shown in the drawing, in a case where a plurality of groove portions 41 are formed, other groove portions which communicate with two or more arbitrary groove portions 41 in the length direction of the transition portion 4 may be formed. In addition, even in a case where a plurality of groove portions 42 are formed, other groove portions which communicate with two or more arbitrary groove portions 42 in the length direction of the transition portion 4 may also be formed.

In the wire 1 of the present invention, forming of the groove portions 41 and 42 suppresses great change in the transverse cross sectional shape from the transition portion 4 over the flat plate portion 5, and therefore, great change in the rigidity is also suppressed. As a result, there is no case where the flat plate portion 5 is twisted when using the wire 1 or is buckled in the vicinity of a boundary between the transition portion 4 and the flat plate portion 5. Therefore, rotary torque of the main body portion 3 is effectively transmitted to the flat plate portion 5. Accordingly, it is possible to make the distal portion of the wire 1 face in intended direction. In addition, a pushing force of the main body portion 3 is effectively transmitted to the flat plate portion 5. As a result, excellent blood vessel followability, pushability, and trackability of the wire 1 are improved.

### <Coil Portion>

As shown in Fig. 1, the coil portion 6 is a coil which is disposed so as to cover the distal side of the core portion 2A and is obtained by forming strands in a spiral shape. The coil may be either a so-called densely wound coil in which adjacent strands are in contact with each other or a coil in which adjacent strands are separated from each other. In addition, the distal side of the coil portion 6 is fixed to the core portion 2A (flat plate portion 5) using the fixation material (fixation portion) 72 or the like.

The materials constituting the strands are not particularly limited, but are preferably metal materials such as stainless steel or Pt-Ni alloy. In addition, the size of the coil portion 6 is not particularly limited, and varies depending on use purpose of the wire 1. In the wire 1 used for PTCA, it is preferable that the coil outer diameter of the coil portion 6 is 0.2 to 0.5 mm and the coil length is 10 to 1000 mm. The coil outer diameter is preferably constant in the length direction of the wire 1, but may be decreased toward the distal side of the wire 1.

The coil portion 6 may be obtained by combining two or more metal materials. For example, the coil portion 6 may include a first coil portion 61 formed of stainless steel strands on the proximal side; and a second coil portion 62 formed of Pt-Ni alloy strands as radiopaque materials on the distal side, and both coil portions 61 and 62 may be joined through welding, adhering, or the like in a boundary portion 63 between the first coil portion 61 and the second coil portion 62. Accordingly, it is easy for the distal side of the wire 1 to be visually checked under X-ray fluoroscopy.

Next, a modification example of the first embodiment of the wire 1 of the present invention will be described.

As shown in Fig. 1, in the wire 1, it is sufficient for the core portion 2A and the coil portion 6 to be fixed to each other in one site on the distal side, but the core portion 2A and the coil portion 6 are preferably fixed to each other in a plurality of sites.

For example, as shown in Fig. 1, in the wire 1, the distal side of the core portion 2A (flat plate portion 5) and the distal side of the coil portion 6 (second coil portion 62) are fixed to each other using the fixation material (fixation portion) 72; a site (the proximal side of the transition portion 4, the small-diameter portion 35, and the distal side of the second tapered portion 34) in the middle of the core portion 2A and a site (boundary portion 63) in the middle of the coil portion 6 are fixed to each other using a fixation material (fixation portion) 73; and a site (the proximal side of the middle-diameter portion 33 and the distal side of the first tapered portion 32) in the middle of the core portion 2A and the proximal side of the coil portion 6 (first coil portion 61) are fixed to each other using a fixation material (fixation portion) 71.

Here, the fixation materials (fixation portions) 71, 72, and 73 are solder (brazing materials), adhesives, or the like. Note that, in the fixation method of the core portion 2A and the coil portion 6, it is not limited to use the fixation materials 71, 72, and 73, and the fixation portions 71, 72, and 73 may be formed through welding.

As shown in Fig. 1, the wire 1 preferably includes a resin covering portion 8 which is formed so as to cover at least the surface of the coil portion 6 on the distal side.

Specifically, the resin covering portion 8 preferably covers a part of the surface of the wire or the entirety of the surface of the wire, that is, the entire surface of the second coil portion 62, the entire surface of the coil portion 6 (the first coil portion 61, the boundary portion 63, and the second coil portion 62), or the entire surface of a site on the proximal side of the coil portion 6 and the core portion 2A.

The resin covering portion 8 is preferably made of resin materials such as a fluorine resin, a maleic anhydride polymeric material, and polyurethane. In addition, the thickness of the resin covering portion 8 is preferably 0.001 to 0.05 mm. Since the wire 1 is covered by such a resin covering portion 8, the frictional resistance (sliding resistance) of the wire 1 is decreased, and the operability in a blood vessel is improved.

Next, a second embodiment of a guide wire according to the present invention will be described.

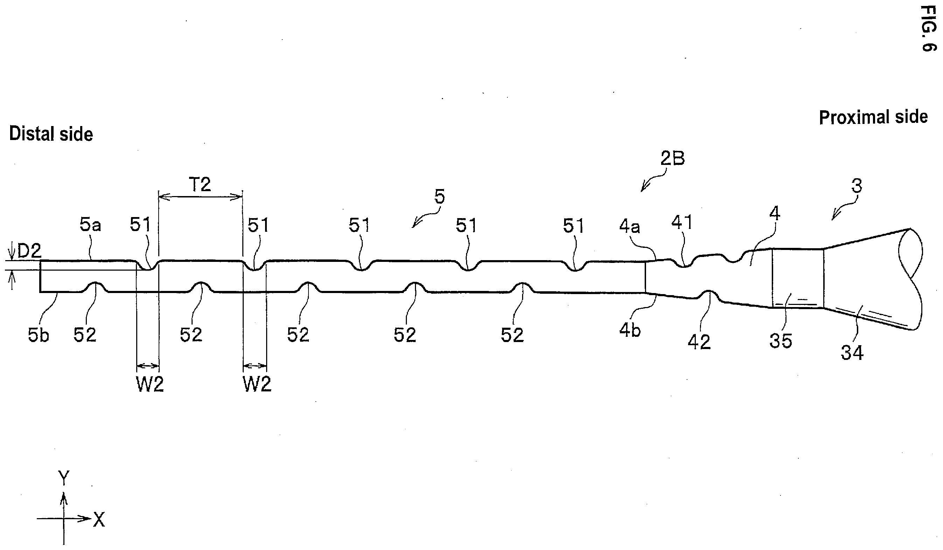

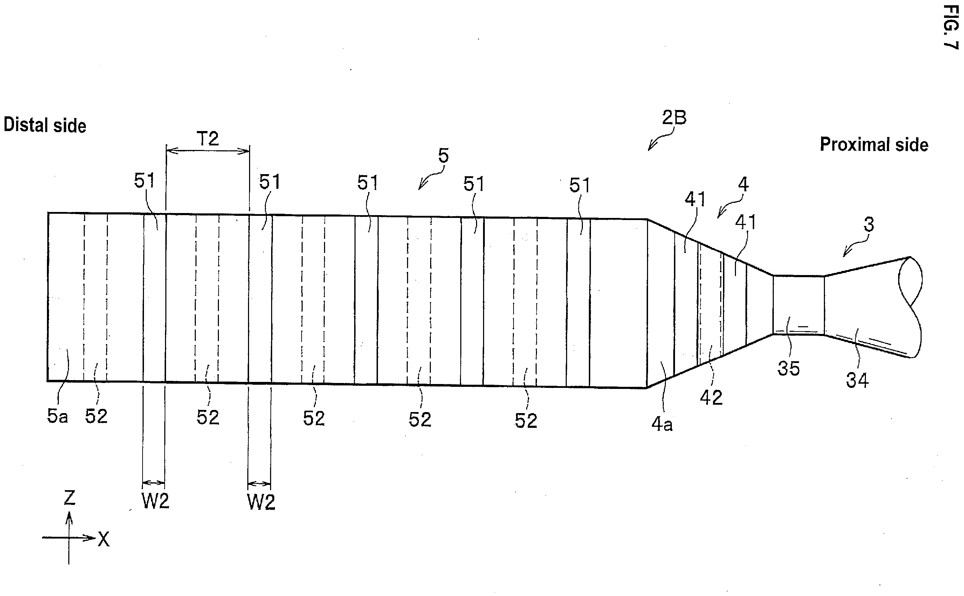

In the guide wire, a core portion 2B (refer to Figs. 6 and 7) is used instead of the core portion 2A (refer to Fig. 1) of the first embodiment. Therefore, at least one of a groove portion 51 or a groove portion 52 extending in a direction different from a length direction is formed in a flat plate portion 5 in the length direction in addition to forming the groove portion 41 or the groove portion 41 and the groove portion 42 in the transition portion 4. In addition, the groove portion 52 may not be formed. The groove portions 51 and 52 are preferably formed by pressing a mold having a surface of a mold, in which convex portions with a shape similar to the groove portions 51 and 52 are formed, on the surface of the flat plate portion 5. Note that the configuration of the first embodiment other than the groove portions 51 and 52 are the same as described above, and therefore, only the groove portions 51 and 52 will be described and the description of other portions will not be repeated.

As shown in Figs. 6 and 7, the groove portion 51 is formed on an upper surface 5a of the flat plate portion 5. Note that, although not shown in the drawing, the groove portion 51 may be formed on a lower surface 5b of the flat plate portion 5. Here, the upper surface 5a and the lower surface 5b are surfaces which become an inner peripheral surface and an outer peripheral surface when a distal portion of the wire is curved. In addition, the direction in which the groove portion 51 is formed is not particularly limited, but is preferably a plate width direction (a direction orthogonal to the length direction) as shown in Fig. 7. Note that, although not shown in the drawing, the groove portion 51 may be formed in an oblique line shape in a direction inclined to the plate width direction at a predetermined angle. Since the oblique line-shaped groove portion 51 is formed in this manner, transmission of rotary torque from a proximal side to a distal side varies depending on the rotational direction of the wire 1 (core portion 2B), and the rotary torque is easily transmitted in the rotational direction opposite to the inclination direction of the oblique line-shaped groove portion 51. As a result, the blood vessel followability of the wire 1 is further improved. Furthermore, the planar shape of the groove portion 51 is preferably a linear shape as shown in Fig. 7, but may be a polygonal line shape or a curved shape.

It is preferable that the transverse cross sectional shape of the groove portion 51 formed in the flat plate portion 5 is an approximately semi-circular shape. However, the transverse cross sectional shape of the groove portion 51 may be other shapes, for example, an approximately U-shape, an approximately V-shape, or an approximately rectangular shape. The number of groove portions 51 is preferably 1 to 500. The groove width W2 of the groove portion 51 is preferably 0.06 to 0.5 mm. The groove depth D2 of the groove portion 51 is preferably 0.001 to 0.03 mm. In addition, in a case of forming a plurality of groove portions 51, the groove width W2 is preferably formed to be constant, but may be formed so as to be increased or decreased toward the distal side. The groove depth D2 is also preferably formed to be constant, but may be formed so as to be increased or decreased toward the distal side.

An interval T2 of adjacent groove portions 51 is preferably 0.1 to 2 mm. The plurality of groove portions 51 are preferably formed at even intervals, but may be formed so that the interval T2 increases or decreases toward the distal side. In addition, the plurality of groove portions 51 may have an interval T2 of 0 mm, that is, may be continuously formed. Furthermore, the groove portions 51 which have been continuously formed may be formed in a part or all of the upper surface 5a or the lower surface 5b of the flat plate portion 5.

As shown in Figs. 6 and 7, in the flat plate portion 5, it is preferable that the groove portion 51 is formed on the upper surface 5a and the groove portion 52 is formed on the lower surface 5b of the flat plate portion 5. In addition, it is preferable that the groove portion 51 and the groove portion 52 are mutually alternately disposed in the length direction of the flat plate portion 5. However, the groove portion 51 and the groove portion 52 may be disposed at the same position as each other. In addition, although not shown in the drawing, in a case where the groove portion 51 is formed on the lower surface 5b, the groove portion 52 is formed on the upper surface 5a.

The formation direction, the planar shape, the transverse cross-sectional shape, the number of grooves, the groove width, and the groove depth of the groove portion 52, and the interval between adjacent groove portions 52 are the same as those of the groove portion 51, and therefore, the description thereof will not be repeated. In addition, the formation direction or the like of the groove portion 52 is preferably the same as that of the groove portion 51, but may be different from that of the groove portion 51.

Although not shown in the drawing, in a case where a plurality of groove portions 51 are formed, other groove portions which communicate with two or more arbitrary groove portions 51 in the length direction of the flat plate portion 5 may be formed. In addition, even in a case where a plurality of groove portions 52 are formed, other groove portions which communicate with two or more arbitrary groove portions 52 in the length direction of the flat plate portion 5 may also be formed.

In the guide wire of the present invention, the rigidity of the flat plate portion 5 is further decreased by forming the groove portions 51 and 52. Therefore, the flexibility of the distal portion of the guide wire is further improved and the risk of perforating a blood vessel is reduced. Thus, the safety is improved. Accordingly, the blood vessel followability of the guide wire is further improved.

Modification examples of the second embodiment of the guide wire of the present invention include an example in which the core portion 2B and the coil portion 6 are fixed to each other in a plurality of sites and an example in which the surface of the wire is covered with the resin covering portion 8, similarly to the first embodiment.

Next, a third embodiment of a guide wire of the present invention will be described.

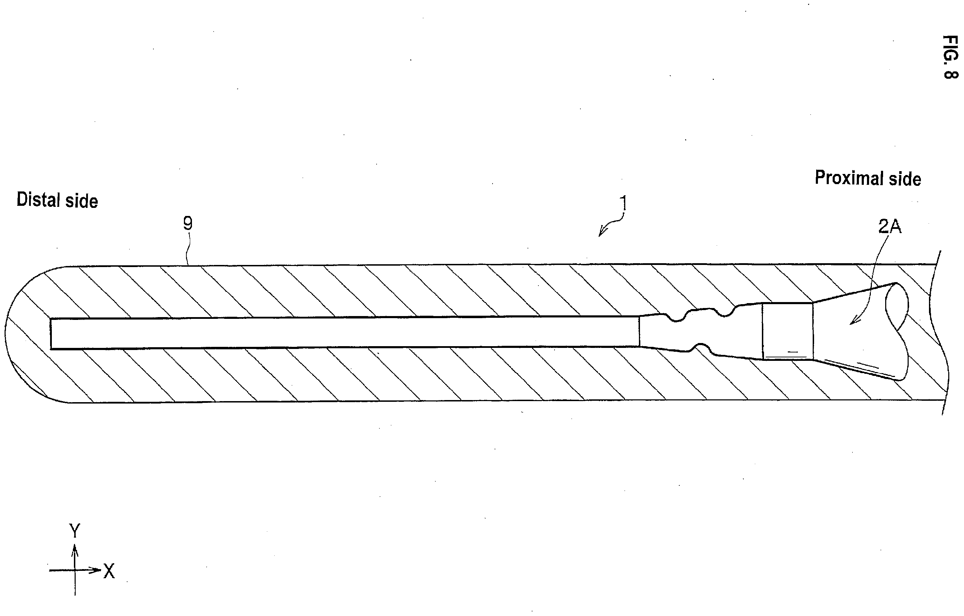

As shown in Fig. 8, a guide wire 1 includes a resin covering portion 9 instead of the coil portion 6 in the first embodiment which includes the core portion 2A. In addition, although not shown in the drawing, the guide wire 1 may include the resin covering portion 9 instead of the coil portion 6 in the second embodiment which includes the core portion 2B (refer to Fig. 6).

Note that the configuration of the first embodiment or the second embodiment other than the resin covering portion 9 is the same as described above. Therefore, only the resin covering portion 9 will be described and the description of other portions will not be repeated.

The resin covering portion 9 is formed so as to cover a distal side of the core portion 2A or the core portion 2B and is made of a resin material. Examples of the resin material include a fluorine resin or polyurethane, and polyurethane is preferable. It is preferable that the resin covering portion 9 is formed such that the thickness thereof on the distal side is thicker than that on the proximal side. The thickness thereof is preferably 10 to 400 µm. In addition, the resin covering portion 9 is preferably formed such that the distal side thereof is rounded.

Since such a resin covering portion 9 is provided, it is prevented that the core portion 2A or the core portion 2B damage a blood vessel wall when using the guide wire 1. Therefore, the safety is improved. In addition, the frictional resistance (sliding resistance) is decreased in the guide wire 1, and therefore, the operability within a blood vessel is also improved.

Next, an exemplary method (not falling under the scope of the claims for using the guide wire of the present invention will be described by taking PTCA for an example.

A distal end of the guide wire is inserted into a femoral artery in a state protruding from a distal end of a guiding catheter, through a Seldinger technique, and is inserted into a right coronary artery via an aorta, an aortic arch, and a right coronary artery orifice. Only the guide wire is made to pass a stenosed site of a blood vessel by being further advanced within the right coronary artery while leaving the guiding catheter at a position of the right coronary artery orifice. Then, the distal end of the guide wire stops at a position beyond the stenosed site of the blood vessel. Accordingly, the passage of a balloon catheter for widening the stenosed site is secured.

Next, a distal end of the balloon catheter, which has been inserted from a proximal side of the guide wire, is made to protrude from the distal end of the guiding catheter, and is inserted into the right coronary artery from the right coronary artery orifice by being further advanced along the guide wire. The distal end of the balloon catheter stops at a position at which a balloon of the balloon catheter reaches a position of the stenosed site of the blood vessel.

Next, the stenosed site of the blood vessel is widened by dilating the balloon after injecting a fluid for dilating a balloon into the balloon catheter from the proximal side thereof. By doing this, deposits such as cholesterol which have been adhered to and deposited in the stenosed site of the blood vessel are physically widened, and therefore, interruption of blood flow is resolved.

The fluid for dilating a balloon is removed from the balloon to deflate the balloon. Next, the balloon catheter, the guide wire, and the guiding catheter are removed from the blood vessel by moving the balloon catheter in the proximal direction along with the guide wire. Accordingly, the procedure of PTCA finishes.

### Reference Signs List

1: guide wire (wire)
2A, 2B: core portion
3: main body portion
4: transition portion
41, 42: groove portion
5: flat plate portion
51, 52: groove portion
6: coil portion
8, 9: resin covering portion

## Claims

1. A guide wire (1) comprising a core portion (2A, 2B) formed of an elongated object having flexibility,
wherein the core portion (2A, 2B) includes a main body portion (3) formed on a proximal side, a flat plate portion (5) formed on a distal side, and a transition portion (4) which connects the main body portion (3) and the flat plate portion (5), and
wherein at least one groove portion (41, 42) extending in a direction different from a length direction is formed on a slope of the transition portion (4) in the length direction, **characterized in that** the transition portion is gradually changed from a circular shape in transverse cross section to a rectangular shape in transverse cross section from the proximal side toward the distal side.

2. The guide wire (1) according to claim 1,
wherein the guide wire (1) includes a coil portion (6) which is disposed so as to cover the distal side of the core portion (2A, 2B) and is obtained by forming strands in a spiral shape, and the core portion (2A, 2B) and the coil portion (6) are fixed to each other on the distal side.

3. The guide wire (1) according to claim 1,
wherein the guide wire (1) includes a resin covering portion (8, 9) which is formed so as to cover the distal side of the core portion (2A, 2B) and is made of a resin material.

4. The guide wire (1) according to any one of claims 1 to 3,
wherein at least one groove portion (51, 52) extending in the direction different from the length direction is formed on at least an upper surface or a lower surface of the flat plate portion (5) in the length direction.

## Patentansprüche

1. Führungsdraht (1), umfassend einen Kernabschnitt (2A, 2B), der aus einem langgestreckten Objekt, das Flexibilität aufweist, gebildet ist,
wobei der Kernabschnitt (2A, 2B) einen Hauptkörperabschnitt (3), der an einer proximalen Seite ausgebildet ist, einen flachen Plattenabschnitt (5), der an einer distalen Seite ausgebildet ist, und einen Übergangsabschnitt (4), welcher den Hauptkörperabschnitt (3) und den flachen Plattenabschnitt (5) verbindet, aufweist, und
wobei mindestens ein Rillenabschnitt (41, 42), der sich in einer Richtung erstreckt, die sich von einer Längsrichtung unterscheidet, an einer Abschrägung des Übergangsabschnitts (4) in der Längsrichtung ausgebildet ist, **dadurch gekennzeichnet, dass** der Übergangsabschnitt allmählich von einer kreisförmigen Form im transversalen Querschnitt zu einer rechteckigen Form im transversalen Querschnitt von der proximalen Seite zur distalen Seite hin geändert wird.

2. Führungsdraht (1) nach Anspruch 1,
wobei der Führungsdraht (1) einen Wicklungsabschnitt (6) umfasst, der so angeordnet ist, dass er die distale Seite des Kernabschnitts (2A, 2B) bedeckt, und der durch das Ausformen von Drähten in einer Spiralform erhalten wird, und der Kernabschnitt (2A, 2B) und der Wicklungsabschnitt (6) an der distalen Seite aneinander befestigt sind.

3. Führungsdraht (1) nach Anspruch 1,
wobei der Führungsdraht (1) einen Harzabdeckungsabschnitt (8, 9) aufweist, der so ausgebildet ist, dass er die distale Seite des Kernabschnitts (2A, 2B) abdeckt, und der aus einem Harzmaterial hergestellt ist.

4. Führungsdraht (1) nach einem der Ansprüche 1 bis 3,
wobei mindestens ein Rillenabschnitt (51, 52), der sich in einer Richtung erstreckt, die sich von einer Längsrichtung unterscheidet, auf mindestens einer oberen Oberfläche oder einer unteren Oberfläche des flachen Plattenabschnitts (5) in der Längsrichtung ausgebildet ist.

## Revendications

1. Un fil de guide (1) comprenant une partie centrale (2A, 2B) formée d'un objet allongé ayant une flexibilité,
dans lequel la partie centrale (2A, 2B) comporte une partie de corps principal (3) formée sur un côté proximal, une partie de plaque plate (5) formée sur un côté distal, et une partie de transition (4) qui connecte la partie de corps principal (3) et la partie de plaque plate (5), et dans lequel au moins une partie de rainure (41, 42) s'étendant dans une direction différente d'une direction de la longueur est formée sur une pente de la partie de transition (4) dans la direction de la longueur,
**caractérisé en ce que** la partie de transition passe progressivement d'une forme circulaire en section transversale à une forme rectangulaire en section transversale du côté proximal vers le côté distal.

2. Le fil de guide (1) selon la revendication 1,
dans lequel le fil de guide (1) comporte une partie de bobine (6) qui est disposée de manière à couvrir le côté distal de la partie centrale (2A, 2B) et est obtenue en formant des brins en forme de spirale, et la partie centrale (2A, 2B) et la partie de bobine (6) sont fixées l'une à l'autre sur le côté distal.

3. Le fil de guide (1) selon la revendication 1,
dans lequel le fil de guide (1) comporte une partie de couverture en résine (8, 9) qui est formée de manière à couvrir le côté distal de la partie centrale (2A, 2B) et est faite d'un matériau en résine.

4. Le fil de guide (1) selon l'une des revendications 1 à 3,
dans lequel au moins une partie de rainure (51, 52) s'étendant dans la direction différente de la direction de la longueur est formée sur au moins une surface supérieure ou une surface inférieure de la partie de plaque plate (5) dans la direction de la longueur.
